# EUROPEAN PATENT APPLICATION

(11) **EP 3 278 775 A1**
(43) Date of publication of application: **07.02.2018**
(21) Application number: 16737101.2
(22) Date of filing: 21.03.2016
(51) Int. Cl.: A61F 5/58

(54) **SPEECH RHYTHM CONTROLLER**

(30) Priority: 03.04.2015 CN 201510158280; 03.04.2015 CN 201520202276 U
(71) Applicant: Hou, Degang, Dalian, Liaoning 116000 (CN)
(72) Inventor: Hou, Degang, Dalian, Liaoning 116000 (CN)
(74) Representative: ProI European Patent Attorneys
(86) International application number: PCT/CN2016/076840
(87) International publication number: WO 2016/112876

(57) **Abstract**

A speech rhythm controller for stuttering correction, comprising: an upper jaw piece and a lower jaw piece. The upper jaw piece comprises: an upper jaw magnetic piece that attaches closely to the inner surface of the maxilla and an upper jaw alloy fixing frame for fixing the upper jaw magnetic piece. The lower jaw piece comprises: a lower jaw magnetic piece that attaches closely to the inner surface of the mandible and a lower jaw alloy fixing frame for fixing the lower jaw magnetic piece. The lower jaw alloy fixing frame and the upper jaw alloy fixing frame are fixed to the outer surface of the teeth to ensure that the upper jaw magnetic piece and the lower jaw magnetic piece are in stably attached to the corresponding maxilla and mandible inner surfaces. Additionally, the upper jaw magnetic piece and the lower jaw magnetic piece are vertically symmetrically arranged, and the sides of the upper jaw magnetic piece and of the lower jaw magnetic piece facing one another have repelling polarity. With the presence of the repelling force between the upper haw magnetic piece and the lower jaw magnetic piece, a fixed resistance must be overcome for the mouth to close, which directly slows the speech speed, ensures one sound per mouth closing, and forms a stable speech rhythm, thereby controlling the speech rhythm, formulating a healthy speech rhythm, and correcting stuttering.

## Description

### TECHNICAL FIELD

The present invention relates to medical device, and more particularly to a speech rhythm controller.

### BACKGROUND ART

Stuttering is caused by human speech rhythm disorders.

Speech correction is the most effective treatment method for speech rhythm disorders (i.e., stuttering). The correction generally includes exercising the speech rhythm of a stutterer through a fixed beat provided by an electronic metronome, or using an auditory delay feedback generator to enable the stutterer to slow down the speed of speech to meet the rhythm of the normal speech. These two correction methods have very long treatment cycle, and the major reason is that the stutterer in a correction mechanism talks with the help of a metronome and also talks with a fixed frequency even if the stutterer does not use the metronome, and therefore it is easy to keep a non-stuttered state. But the patient generally does not stay always in the correction mechanism, this correction treatment is generally carried out within a period of time every day, and then the patient continues to normal life. The patient will stutter again after leaving from this environment, which leads a slow treatment.

At present there is no equipment that not only does not affect the daily life of stutterers, but also has an effect of controlling the speech rhythm of the stutterers.

### SUMMARY OF THE INVENTION

With respect to the above technical problem, the present invention provides a speech rhythm controller which can control a speech rhythm of a stutterer without affecting the daily life of the stutterer.

To achieve the said purpose, the present invention is realized by the following technical solution:
a speech rhythm controller comprises an upper jaw piece and a lower jaw piece; wherein the upper jaw piece comprises an upper jaw magnetic piece that attaches closely to the inner surface of the maxilla, and an upper jaw alloy fixing frame for fixing the upper jaw magnetic spiece; the upper jaw alloy fixing frame is of a metal wire braid structure and fixed to the outer surfaces of teeth of the maxilla, and two ends of the upper jaw alloy fixing frame are fixed to the upper jaw magnetic piece fitted with the inner surface of the maxilla after bypassing respectively the tail end at the left side of an upper jaw gum and the tail end at the right side of the upper jaw gum; the lower jaw piece comprises a lower jaw magnetic piece that attaches closely to the inner surface of the mandible and a lower jaw alloy fixing frame for fixing the lower jaw magnetic piece; the lower jaw alloy fixing frame is of a metal wire braid structure and fixed to the outer surfaces of teeth of the mandible, and two ends of the lower jaw alloy fixing frame are fixed to the lower jaw magnetic piece fitted with the inner surface of the mandible after bypassing respectively the tail end at the left side of a lower jaw gum and the tail end at the right side of the lower jaw gum.

The upper jaw magnetic piece and the lower jaw magnetic piece are arranged oppositely up and down, and the opposite surfaces of the upper jaw magnetic piece and of the lower jaw magnetic piece facing one another have repelling polarity.

The upper jaw alloy fixing frame and the upper jaw magnetic piece are detachably connected; the lower jaw alloy fixing frame and the lower jaw magnetic piece are detachably connected also.

According to the present invention adopting the above technical solution, the upper jaw magnetic piece is fixed to the outer surfaces of the teeth of the upper jaw through the upper jaw alloy fixing frame to ensure the upper jaw magnetic piece keeps fitting with the oral cavity upper jaw. The lower jaw magnetic piece is fixed to the outer surfaces of the teeth of the lower jaw through the lower jaw alloy fixing frame to ensure the lower jaw magnetic piece keeps fitting with the oral cavity lower jaw. In addition, because the upper jaw magnetic piece and the lower jaw magnetic piece repel each other, it is necessary to overcome a fixed resistance when the oral cavity is closed so as to delay the speed of speech directly and ensure one closing action of the oral cavity is taken as one sound, and therefore a stable speech rhythm is formed. As time passes, neurological reflex and muscle memory will be produced, so that the habit according with the normal speech rhythm is formed, and then a stutterer will not stutter.

The upper jaw alloy fixing frame and the lower jaw alloy fixing frame are made of medical alloy, and a dental band commonly used in a dental hoop can be arranged on the fitted parts of the upper jaw alloy fixing frame fitting to the outer surfaces of the teeth as well as the fitted parts of the lower jaw alloy fixing frame fitting to the outer surfaces of the teeth to realize further fixation, or the upper jaw alloy fixing frame and the lower jaw alloy fixing frame may be directly designed into a dental hoop form.

The present invention has the following advantages:
1. the speech rhythm controller is directly arranged inside the oral cavity and has a good fitting state without foreign body sensation;
2. the speech rhythm controller can provide a fixed and constant resistance to ensure the generation of a stable speech rhythm habit;
3. The repulsion force can be changed by adjusting the sizes of the upper jaw magnetic piece and the lower jaw magnetic piece according to different crowds, such as adults and children;
4. the alloy fixing frames can be detached from the magnetic pieces to facilitate adjustment.

The foregoing description is merely an overview of the technical solution of the present invention. In order to enable a clearer understanding of the technical means of the present invention, and implement it in accordance with the content of the present invention, and make the foregoing and other objects, features and advantages of the present invention more apparent, the preferred embodiments will be illustrated and described in detail below in conjunction with the accompanying drawings.

### BRIEF DESCRIPTION OF THE DRAWINGS

There are five drawings totally in the present invention, wherein:
Fig. 1 is a side-view stereoscopic structural schematic drawing of an upper jaw piece of the present invention.
Fig. 2 is a back-view stereoscopic structural schematic drawing of the upper jaw piece of the present invention.
Fig. 3 is a top-view structural schematic drawing of the upper jaw piece of the present invention.
Fig. 4 is a side-view stereoscopic structural schematic drawing of a lower jaw piece of the present invention.
Fig. 5 is a stereoscopic structural schematic drawing in which the speech rhythm controller is fixed in an oral cavity according to the present invention.

### DETAILED DESCRIPTIONS OF THE PREFERRED EMBODIMENTS

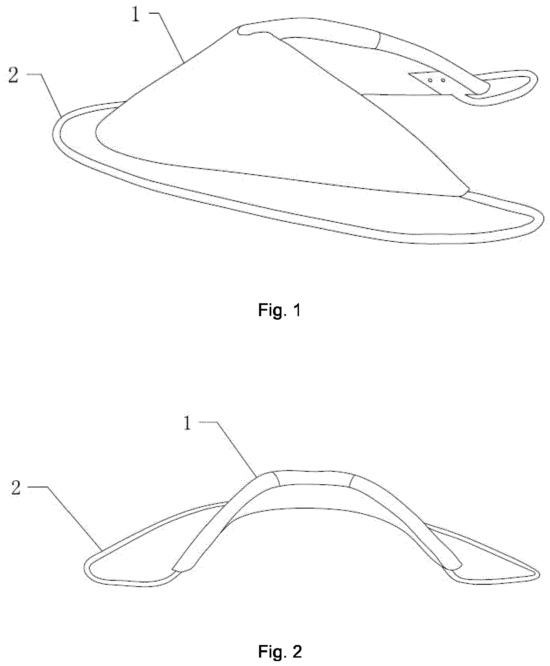

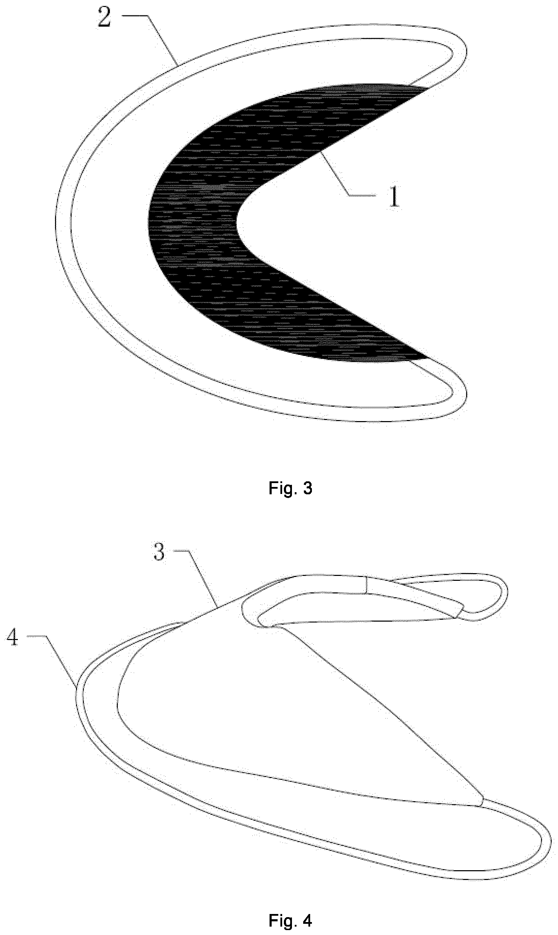

A speech rhythm controller as shown in Figs. 1, 2, 3 and 4 comprises an upper jaw piece and a lower jaw piece;
the upper jaw piece comprises an upper jaw magnetic piece 1 that attaches closely to the inner surface of the maxilla, and an upper jaw alloy fixing frame 2 for fixing the upper jaw magnetic piece 1;
the upper jaw alloy fixing frame 2 is of a metal wire braid structure and fixed to the outer surfaces of teeth of the maxilla, and two ends of the upper jaw alloy fixing frame 2 are fixed to the upper jaw magnetic piece 1 fitted with the inner surface of the maxilla after bypassing the tail end at the left side of an upper jaw gum and the tail end at the right side of the upper jaw gum respectively;
the lower jaw piece comprises a lower jaw magnetic piece 3 that attaches closely to the inner surfaces of the mandible, and a lower jaw alloy fixing frame 4 for fixing the lower jaw magnetic piece 3;
the lower jaw alloy fixing frame 4 is of a metal wire braid structure and fixed to the outer surfaces of teeth of the mandible, and two ends of the lower jaw alloy fixing frame 4 are fixed to the lower jaw magnetic piece 3 fitted with the inner surface of the mandible after bypassing the tail end at the left side of a lower jaw gum and the tail end at the right side of the lower jaw gum respectively.

The upper jaw magnetic piece 1 and the lower jaw magnetic piece 3 are arranged oppositely up and down, and the opposite surfaces of the upper jaw magnetic piece 3 and of the lower jaw magnetic piece 4 facing one another having repelling polairty;
wherein, the upper jaw alloy fixing frame 2 and the upper jaw magnetic piece 1 are detachably connected; the lower jaw alloy fixing frame 4 and the lower jaw magnetic piece 3 are detachably connected also.

According to the present invention adopting the above technical solution, the upper jaw magnetic piece 1 is fixed to the outer surfaces of the teeth of the upper jaw through the upper jaw alloy fixing frame 2 to ensure the upper jaw magnetic piece 1 keeps fitting with the oral cavity upper jaw. The lower jaw magnetic piece 3 is fixed to the outer surfaces of the teeth of the lower jaw through the lower jaw alloy fixing frame 4 to ensure the lower jaw magnetic piece 3 keeps fitting with the oral cavity lower jaw. In addition, because the upper jaw magnetic piece 1 and the lower jaw magnetic piece 3 repel each other, it is necessary to overcome a fixed resistance when the oral cavity is closed so as to delay the speed of speech directly and ensure one closing action of the oral cavity is taken as one sound, and therefore a stable speech rhythm is formed. As time passes, neurological reflex and muscle memory will be produced, so that the habit according with the normal speech rhythm is formed, and then a stutterer will not stutter.

The upper jaw alloy fixing frame 2 and the lower jaw alloy fixing frame 4 are made of medical alloy, and a dental band commonly used in a dental hoop can be arranged on fitted parts of the upper jaw alloy fixing frame 2 fitting to the outer surfaces of the teeth as well as the fitted parts of the lower jaw alloy fixing frame 4 fitting to the outer surfaces of the teeth to realize further fixation, or the upper jaw alloy fixing frame and the lower jaw alloy fixing frame may be directly designed into a dental hoop form.

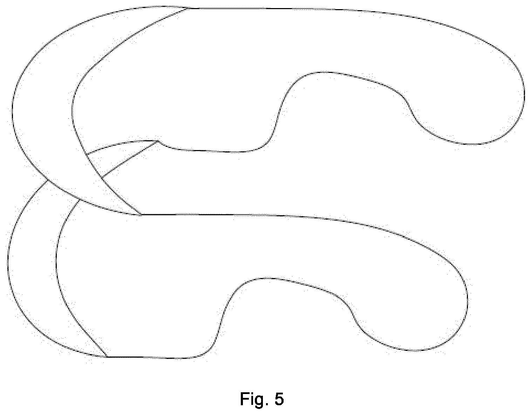

The upper jaw piece and the lower jaw piece may be integrally designed as shown in Fig. 5. In this case, the upper jaw piece and the lower jaw piece are located inside the oral cavity, two sides of each of the magnetic pieces are supported and fixed by the corresponding alloy frame. The alloy frames at two sides are arranged respectively adjacent to the inner sides of the left gum and the right gum, such that the two magnetic pieces are still fitted with the upper jaw inner surface and the lower jaw inner surface respectively.

The foregoing contents are merely preferred embodiments of the present invention and are not intended to limit the present invention in any form. Although the present invention has been described with reference to the preferred embodiments, they are not intended to limit the present invention, and any person skilled in the art will be able to make a slight change or modification as equivalently-varied equivalent embodiment using the technical content disclosed by the present invention. Any simple amendments, equivalent changes and modifications made to the above embodiments in accordance with the technical content disclosed by the present invention, without departing the content of the technical solution, still fall within the scope of the technical solution of the present invention.

## Claims

1. A speech rhythm controller, comprising an upper jaw piece and a lower jaw piece;
wherein the upper jaw piece comprises an upper jaw magnetic piece (1) that attaches closely to the inner surface of the maxilla and an upper jaw alloy fixing frame (2) for fixing the upper jaw magnetic piece (1);
the upper jaw alloy fixing frame (2) is of a metal belt structure and fixed to the outer surfaces of teeth of the maxilla, and two ends of the upper jaw alloy fixing frame (2) are fixed to the upper jaw magnetic piece (1) fitted with the inner surface of the maxilla after bypassing respectively the tail end at the left side of an upper jaw gum and the tail end at the right side of the upper jaw gum;
the lower jaw piece comprises a lower jaw magnetic piece (3) that attaches closely to the inner surface of the mandible and a lower jaw alloy fixing frame (4) for fixing the lower jaw magnetic piece (3);
the lower jaw alloy fixing frame (4) is of a metal belt structure and fixed to the outer surfaces of teeth of the mandible, and two ends of the lower jaw alloy fixing frame (4) are fixed to the lower jaw magnetic piece (3) fitted with the inner surface of the mandible after bypassing respectively the tail end at the left side of a lower jaw gum and the tail end at the right side of the lower jaw gum;
wherein, the upper jaw magnetic piece (1) and the lower jaw magnetic piece (3) are arranged oppositely up and down, and the opposite surfaces of the upper jaw magnetic piece (3) and of the lower jaw magnetic piece (4) facing one another have repelling polarity.
